(19) Europäisches Patentamt
European Patent Office
Office européen des brevets

(11) Veröffentlichungsnummer: **0 196 628**
**B1**

(12) **EUROPÄISCHE PATENTSCHRIFT**

(45) Veröffentlichungstag der Patentschrift: **04.07.90**

(21) Anmeldenummer: **86104233.1**

(22) Anmeldetag: **27.03.86**

(51) Int. Cl.[5]: **C 07 D 225/08,** C 12 P 17/10, A 61 K 31/395 // (C12P17/10, C12R1:465)

# (54) Ein neues Ansamycin-Antibiotikum, ein mikrobielles Verfahren zu seiner Herstellung und seine Verwendung als Arzneimittel.

(30) Priorität: **03.04.85 DE 3512194**

(43) Veröffentlichungstag der Anmeldung:
**08.10.86 Patentblatt 86/41**

(45) Bekanntmachung des Hinweises auf die Patenterteilung:
**04.07.90 Patentblatt 90/27**

(84) Benannte Vertragsstaaten:
**AT BE CH DE FR GB IT LI NL**

(56) Entgegenhaltungen:
**THE JOURNAL OF ANTIBIOTICS, Band 38, Nr. 7, Juli 1985, Seiten 948-951; T. MUKHOPADHYAY et al.: "A new ansamycin antibiotic, naphthomycin H from a Streptomyces species"**

**CHEMICAL ABSTRACTS, Band 107, 28. September 1987, Zusammenfassung Nr. 114267a, Columbus, Ohio, US**

(73) Patentinhaber: **HOECHST AKTIENGESELLSCHAFT
Postfach 80 03 20
D-6230 Frankfurt am Main 80 (DE)**

(72) Erfinder: **Franco, Christopher Milton Mathew, Dr.
74A The Westminister Duncan Causeway
Sion Bombay 400 022 (IN)**
Erfinder: **Reddy, Goukanapalli Chandra Sekhara, Dr.
Flat 104, Aarti Apartments Sarojini Naidu Road
Mulund (West) Bombay 400 080 (IN)**
Erfinder: **Mukhopadhyay, Triptikumar, Dr.
15, A-Wing Hansa Sagar Gvardhan Nagar
LBS Marg, Mulund West Bombay 400080 (IN)**
Erfinder: **Ganguli, Bimal Naresh, Dr.
Saurayuth 173 Central Avenue
Chembur Bombay 400 071 (IN)**
Erfinder: **Fehlhaber, Hans-Wolfram, Dr.
Thomas-Mann-Strasse 5a
D-6270 Idstein (DE)**

**EP 0 196 628 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Die vorliegende Erfindung betrifft ein neues Ansamycin-Antibiotikum mit der Bezeichnung Naphthomycin H sowie ein Verfahren zu seiner Herstellung aus einem Micromonospora mit der Bezeichnung *Streptomyces* Y—8340369 (DSM 3278).

Ansamycin-Antibiotika werden bekanntlich von verschiedenen Species von Streptomyces, Nocordia und Micromonspora erzeugt. Über Ansamycin-Antibiotika aus der Familie der Naphythomycine wurde berichtet, daß sie von verschiedenen Streptomyces-Arten produziert werden. Sie sind auch in der Literatur beschrieben. Ansamycin-Antibiotika spielen eine bedeutende Rolle in der Medizin, als antibakterielle Mittel, bei der Bekämpfung von Tumoren in der Krebstherapie und können auch in der Landwirtschaft zur Bekämpfung von phytopathogenen Pilzen eingesetzt werden. Bisher wurden verschiedene Ansamycine beschrieben, darunter Rifamycin, das in der Klinik bei der Tuberkulosebehandlung angewendet wurde.

Ansamycine sind in Antibiot. Ann 1958—60, 262 (1960); Pure Appl. Chem. *7*, 551 (1963), Fortschr. Chem. Org. Naturst. *33*, 231—307 (1976); J. Amer. Chem. *92*, 7591 (1970), *93*, 6275 (1971); J. Antibiotics 23, 442 (1970) und *24*, 810 (1971); Accounts of Chemical Research *5*, 57 (1972); Helv. Chim. Acta *56*, 2279—2287 (1973); Topics in Current Chemistry *72*, 21—49 (1977) und in "Topics in Antibiotic Chemistry", Band I, S. 93—217, Sammes P. G. (Herausgeber), London (1977), beschrieben.

Rifamycine sind beschrieben in Farmace Ed. Sci. *14*, 146 (1959); *15*, 228 (1960) und *16*, 165 (1961); Prog. Indust. Microbiol. *6*, 21 (1967); Arzneimittel-Forsch. *21*, 1907 (1971) und den U.S.-Patenten 2 999 048, 3 884 763 und 4 013 789.

Tolypomycine sind beschrieben in den J. Antibiotics *31*, 1195 (1975) und *25*, 11 (1972) und im Deutschen Patent 2 015 076.

Halomycine sind beschrieben in Antimicrobial Agents and Chemotherapy *1967*, 435—441 und J. Antibiotics *30*, 625 (1977).

Streptovaricine sind beschrieben in Ann. Rev. Tuberc. Pulm. Dis. *75*, 576—583, (1957); J. Antibiotics *21*, 204 (1968) und *25*, 71—73 (1972); und Amer. Chem. Soc. *93*, 6273 (1971).

Unter den Naphthomycin-Antibiotika sind das Naphthomycin A in Arch. Mikrobiol. *65*, 303—317 (1969); J. Antibiotics *28*, 85—86 (1975) und *32*, 167 (1979) und die Naphthomycine B und C im J. Antibiotics *36*, 484—492 (1983) beschrieben. Das Actamycin ist in den Tetrahed. Lett. *22*, 1145—1148 (1981) und *22*, 1149—1152 (1981) beschrieben.

Eine Zusammenfassung über Ansamycin-Antibiotika findet sich weiterhin im "Index of Antibiotics from Actinomycetes", Hamao Umezawa (Hauptherausgeber) Band I, S. 218, 564—566 und 624, Univ. Park Press, State College, Pennsylvania, USA (1967) und Band II, S. 414, 446—450, 735, 897—905, 984—985, 952—954, 995—996 und 1116, Univ. Park Press Baltimore, USA (1978).

Ein Index über Ansamycine findet sich weiterhin auf den Seiten 451—491 des "CRS Handbook of Antibiotic Compounds", Band II, Microcyclic Lactone (Lactam) Antibiotics, James Berdy (Verfasser), CRC Press Inc. Boca Raton, Florida, USA (1980).

Das Lehrbuch "Antibiotics", Band III, herausgegeben von J. W. Corceran und Fred. E. Hahn, Springer-Verlag, New York, USA (1975) gibt einen Überblick über "Rifomycine and Other Ansamycins" von W. Wehrli und M. Staehlin auf den Seiten 252—268, während im Band IV, herausgegeben von J. W. Corceran, Springer-Verlag, New York, USA, (1981) ein Überblick über die "Biosynthesis of Ansamycins" von G. Lancini und M. Grandi, S. 12—40 zu finden ist.

Gegenstand der vorliegenden Erfindung ist ein neues Ansamycin mit der Bezeichnung Naphthomycin H sowie ein Verfahren zu seiner Herstellung aus einem Mikroorganismus mit der Bezeichnung *Streptomyces* Y—8340369 (DSM 3278).

Der erfindungsgemäßen Verbindung konnte die folgende Strukturformel I zugeordnet werden:

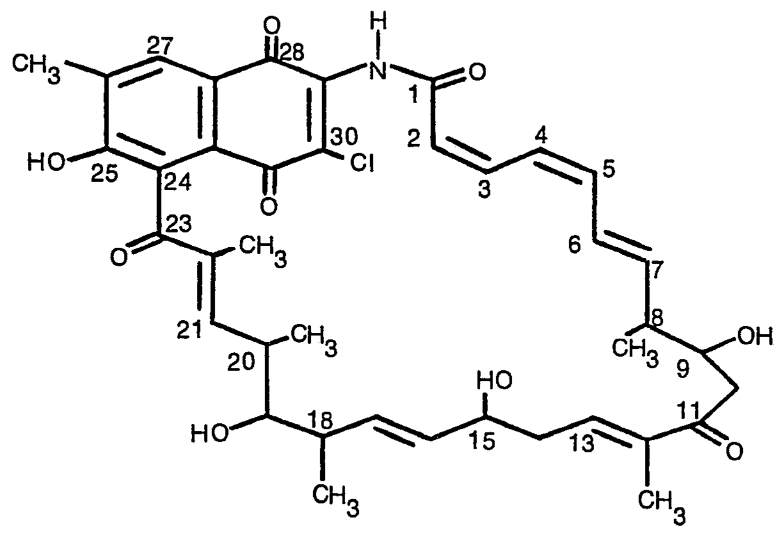

Der Mikroorganismus zur Produktion von Naphthomycin H wurde als eine *Streptomyces*-Art aus der Ordnung *Actinomycetales* aus der Familie Streptomycetaceae und der Gattung *Streptomyces* identifiziert. In der vorliegenden Erfindung wird der Mikroorganismus als *Streptomyces* echinatus Y—8340369 beschrieben. Es wurde am 22.3.1985 bei der Deutschen Sammlung von Mikroorganismen unter der Aufnahme-Nr. 3278 hinterlegt.

Das Verfahren zur Herstellung des neuen Ansamycin-Antibiotikums mit der Bezeichnung Naphthomycin H ist dadurch gekennzeichnet, daß Streptomyces Y—8340369 (DSM 3278) durch Fermentation unter aerobischen Bedingungen in einem Nährmendium, das Kohlenstoff- und Stickstoffquellen, mineralische Nährsalze und Spurenelemente enthält, gezüchtet wird und das dabei gebildete Antibiotikum auf bekannte Art und Weise, wie im folgenden beschrieben, aus der Kulturbrühe isoliert und gereinigt wird.

Als Kohlenstoffquellen eigenen sich Glukose, Rohrzucker, Stärke, Glycerin, Dextrin, Fruchtzucker, Melasse, Hafermehl, Malzzucker, Milchzucker oder Galaktose, vorzugsweise Glukose, Stärke, Dextrin und Mannit. Als Stickstoffquellen können Sojabohnenmehl, Hefemehl, Hefeextrakt, Fleischextrakt, Malzextrakt, Maisquellwasser, Pepton, Casein, Baumwollsaatöl oder anorganische Substanzen wie Ammoniumsalz oder Nitrate (z.B. Ammoniumsulfat, Natriumnitrat, Ammonimchlorid oder Kaliumnitrat) verwendet werden. Vorzugsweise wird Malzextrakt, Hefeextrakt, Sojabohnenmehl, Pepton und Maisquellwasser ein gesetzt. Als anorganische Nährsalze eignen sich Natriumchlorid, Magnesiumsulfat, Kaliumchlorid, Kaliumhydrogenphosphat und Kaliumdihydrogenphosphat, Calciumchlorid, Calciumcarbonat, Ammoniumhydrogenphosphat, Natriumhydrogenphosphat und Natriumdihydrogenphosphat, Magnesiumphosphat, oder Calciumphosphat. Als Spurenelemente können Eisen-, Mangan-, Kupfer-, Zink- oder Kobaltsalze oder Salze anderer Schwermetalle verwendet werden.

Die Kultivierung von Streptomyces Y—8340369 kann gegebenenfalls in Anwesenheit eines oder mehrerer Schaumverhütungsmittel(s) wie Silicon oder Desmophen® durchgeführt werden.

Die Kultivierung von *Streptomyces* Y—8340369 kann bei Temperaturen von 24—40°C bei einem pH-Wert von 6,0 bis 8,0, vorzugsweise unter aerobischen Bedingungen bei 27°C und pH 6,8, erfolgen. Die Fermentation wird nach 40—66 Stunden abgebrochen, wenn die Ausbeute an der erfindungsgemäßen Verbindung optimal ist. Vorzugsweise kann es sich bei der Fermentierung um eine Submersfermentierung handeln. Das Schaumverhütungsmittel kann zu Beginn der Fermentierung zugesetzt werden, so daß es in der Kulturbrühe in eine Konzentration von 0,025% enthalten ist.

Der Fortschritt der Fermentation und die Bildung des Naphthomycins H der vorliegenden Erfindung können durch Messen der antibakteriellen Wirksamkeit der Kulturbrühe gegen *Staphylococcus aureus* 209P verfolgt werden.

Das Naphthomycin H befindet sich in der erhaltenen Kulturbrühe sowohl im Mycel als auch im Kulturfiltrat.

Das Naphthomycin H wird auf bekannte Art und Weise Aus der Kulturbrühe isoliert und gereinigt. So kann Naphthomycin H aus dem Kulturfiltrat mittels eines wasserunlöslichen Lösungsmittels wie Essigsäureethylester, Chloroform oder Butanol nach Einstellen des pH-Wertes des Filtrats auf 6,5—7,5 extrahiert werden. Als Lösungsmittel bevorzugt man Essigsäureethylester und als pH-Wert einen pH von 7,0. Aus dem Mycel wird die erfindungsgemäße Verbindung durch Extraktion des durch Filtrieren oder Zentrifugieren gewonnenen Mycels mit Lösungsmitteln wie Essigsäureethylester, Chloroform, Methanol, Ethanol, Aceton, Butanol, oder Methylethylketon oder mit sauren Lösungen wie Salzsäurelösung oder Essigsäurelösung extrahiert. Bevorzugtes Lösungsmittel ist Aceton. Nach der Extraktion wird das Lösungsmittel beispielsweise durch Verdampfen im Vakuum entfernt, die wäßrige Schicht auf pH 6,5—7,5 eingestellt und mit einem Lösungsmittel wie Essigsäureethylester extrahiert. Vorzugsweise wird der pH-Wert auf 7,0 eingestellt. Die Lösungsmittelextrakte des Kulturfiltrats und des Mycels werden vereinigt, zur Trockene eingedampft und beispielsweise säulenchromatographisch gereinigt.

Zur Isolierung des Naphthomycins H kann die Kulturbrühe auch als solche der beim Mycel angewandten Lösungmittelextraktion ohne vorherige Abtrennung des Mycels ausgesetzt werden.

Eine andere Methode, Naphthomycin H aus der Kulturbrühe zu gewinnen, basiert auf der Adsorption. Dabei wird die flüssige Substanz, beispielsweise das Kulturfiltrat oder die die erfindungsgemäße Verbindung enthaltenden Lösungsmittelextrakte säulenchromatographisch oder flüssigkeitschromatographisch etc. unter Verwendung geeigneter Adsorptionsmittel wie Aktivkohle, Diaion HP—20®, XAD®, Tonerde, Kieselgel oder Sephadex LH—20® behandelt. Die erfindungsgemäße Verbindung wird aus den Adsorptionsmitteln mit entsprechenden Laufmitteln wie Methaol oder Aceton eluiert, die Eluate werden zur Trockene eingeengt und beispeilsweise säulenchromatographisch gereinigt. Gegebenenfalls kann die Reinigung auch mittels Gegenstromverteilung, präparativer Dünnschichtchromatographie und Kristallisation erfolgen. Die weitere Reinigung kann durch Hochdruck-Flüssigkeitschromatographie erfolgen.

Die Erfindung wird durch die nachfolgenden Beispiele näher erläutert:

Beispiel I

Kultivierung von Streptomyces Y—8340369 (DSM 3278) zur fermentativen Herstellung von Naphthomycin H

*Streptomyces* Y—8340369 (DSM 3278) wurde auf ein Hefe-Malz-Agar Nährmedium der folgenden Zusammensetzung gegeben:

| | |
|---|---|
| Malzextrakt | 10,0 g |
| Hefeextrakt | 4,0 g |
| Glucose | 4,0 g |
| Agar | 15,0 g |
| Destilliertes Wasser | 1 l |
| pH | 7,0 |

Das Medium wurde auf Teströhrchen verteilt und 20 Minuten bei 121°C sterilisiert. Dann wurden die Röhrchen in Schräglage zur Herstellung von Agar-Schrägröhrchen abgekühlt, mit der aus dem Boden isolierten *Streptomyces* Y—8340369-Kultur beimpft und 10—15 Tage bei 28°C inkubiert, bis ein gutes Wachstum und eine gute Sporenbildung festgestellt wurden. Fünf 500 ml-Erlenmeyer-Kolben mit je 100 ml Impfmedium bzw. eine 5 l Saugflasche mit 1 l Impfkulturmedium wurden bzw. wurde dann mit einer Sporensuspension in destilliertem Wasser aus einem Schrägröhrchen beimpft.

Zusammensetzung des Impfkulturmediums:

| | |
|---|---|
| Glucose | 15,0 g |
| Sojabohnenmehl | 15,0 g |
| Maisquellwasser | 5,0 g |
| $CaCO_3$ | 2,0 g |
| NaCl | 5,0 g |
| Destilliertes Wasser | 1 l |
| pH | 7,0 |

Dann verteilte man je 100 ml Impfkulturmedium auf 500 ml -Erlenmeyerkolben bzw. 1 l Impfkulturmedium in eine 5 1-Saugflasche und sterilisierte 20 Minuten bei 120°C. Die Kolben bzw. die Flasche wurde(n) abgekühlt, mit der Sporensuspension beimpft und bei 240 UpM 72 Stunden bei 28°C in einem Rotationsschüttelapparat mit einer Ampflitude von 3,75 cm geschüttelt. Die gewachsene Kultur wurde als Inokulum für einen 15 1-Glasfermenter mit 10 l eines 10 vol.%-igen Impfkulturmediums zur Herstellung einer Impfkultur für die zweite Stufe benutzt. Die Fermentation wurde bei 27°C (±1°C) unter Rühren bei 160—180 UpM bei einer Belüftungsrate von 0,6—0,8 VVM 25 Stunden lang durchgeführt. Die dabei erhaltene, gut gewachsene Zweitstufenimpfkultur wurde als Inokulum für das Produktionsmedium verwendet.

Zusammensetzung des Produktionsmediums

| | |
|---|---|
| Stärke | 10,0 g |
| Glucose | 10,0 g |
| Malzextrakt | 7,5 g |
| Pepton | 3,0 g |
| NaCl | 1,0 g |
| $MgSO_4 \cdot 7\ H_2O$ | 1,0 g |

| | |
|---|---|
| $CuSO_4 \cdot 5\ H_2O$ | 7,0 mg |
| $FeSO_4 \cdot 7\ H_2O$ | 1,0 mg |
| $MnCl_2 \cdot 4\ H_2O$ | 8,0 mg |
| $ZnSO_4 \cdot 7\ H_2O$ | 2,0 mg |
| Destilliertes Wasser | 1 l |
| pH | 7,0 |

Die Ansätze der Fermenter wurden mit 0,025% Desmophen® versetzt. 100 l des Produktionsmediums füllte man in einen 150 l Fermenter. Das Medium wurde durch indirekten und durch direkten Dampf 28 Minuten bei 121°C sterilisiert. Der Fermenter wurde abgekühlt und mit der Zweitstufenimpfkultur (10 V/V%) beimpft. Die Fermentation wurde 66 Stunden bei 27°C (± 0,5°C) unter Rühren bei 120—140 UpM bei einer Belüftungsrate von 0,6—0,8 VVM durchgeführt. Beim Abbruch der Fermentation nach 66 Stunden betrug der pH-Wert der Kulturbrühe 6,44 und das abgesetzte Zellvolumen 18 ml auf 100 ml.

Beispiel II

Kultivierung von *Streptomyces* Y—8340369 (DSM 3278) zur fermentativen Herstellung von Naphthomycin H

Wie Beispiel I bis auf die folgenden Unterschiede:

Zusammensetzung des Agar-Mediums

| | |
|---|---|
| Hafermehl | 20,0 g |
| Agar | 15,0 g |
| $FeSO_4 \cdot 7\ H_2O$ | 0,1 mg |
| $ZnSO_4 \cdot 7H_2O$ | 0,1 mg |
| $MnCl_2 \cdot 4H_2O$ | 0,1 mg |
| Destilliertes Wasser | 1 l |
| pH | 7,2 |

Zusammensetzung des Produktionsmediums

| | |
|---|---|
| Stärke | 10,0 g |
| Glucose | 10,0 g |
| Sojabohnenmehl | 15,0 g |
| $K_2HPO_4$ | 1,0 g |
| $MgSO_4 \cdot 7\ H_2O$ | 1,0 g |
| NaCl | 3,0 g |
| $CuSO_4 \cdot 5\ H_2O$ | 7,0 mg |
| $FeSO_4 \cdot 7\ H_2O$ | 1,0 mg |
| $MnCl_2 \cdot 4\ H_2O$ | 8,0 mg |
| $ZnSO_4 \cdot 7\ H_2O$ | 2,0 mg |
| Destilliertes Wasser | 1 l |
| pH | 7,0 |

Beim Abernten des Fermenters betrug der pH-Wert der Kulturbrühe 6,35 und das abgesetzte Zellvolumen 25 ml auf 100 ml.

Beispiel III

Kultivierung von *Streptomyces* Y—8340369 (DSM 3278) zur fermentativen Herstellung von Naphthomycin H

Wie Beispiel I bis auf die folgenden Unterschiede:

Zusammensetzung des Kulturmediums

| | |
|---|---|
| Stärke | 30,0 g |
| Rohrzucker | 10,0 g |
| Glucose | 10,0 g |
| Sojapepton | 15,0 g |
| Maisquellwasser | 10,0 g |
| $K_2HPO_4$ | 3,0 g |
| NaCl | 1,0 g |
| $CaCO_3$ | 3,0 g |
| $CuSO_4 \cdot 5\ H_2O$ | 7,0 mg |
| $FeSO_4 \cdot 7\ H_2O$ | 1,0 mg |
| $MnCl_2 \cdot 4\ H_2O$ | 8,0 mg |
| $ZnSO_4 \cdot 7\ H_2O$ | 2,0 mg |
| Destilliertes Wasser | 1 l |
| pH | 7,2 |

Zusammensetzung des Produktionsmediums

| | |
|---|---|
| Dextrin | 20,0 g |
| Sojabohnenmehl | 10,0 g |
| Hefeextrakt | 2,0 g |
| $FeSO_4 \cdot 7\ H_2O$ | 0,1 g |
| Destilliertes Wasser | 1 l |
| pH | 7,0 |

Beim Abernten des Fermenters betrug der pH-Wert der Kulturbrühe 6,73 und das abgesetzte Zellvolumen 24 ml auf 100 ml.

Beispiel IV

Kultivierung von *Streptomyces* Y—8340369 (DSM 3278) zur fermentativen Herstellung von Naphthomycin H

Wiw Beispiel I bis auf die folgenden Unterschiede:

Zusammensetzung der Impfkulturmediums:

| | |
|---|---|
| Glucose | 30,0 g |
| Sojabohnenmehl | 30,0 g |

| | |
|---|---|
| Maisquellwasser | 10,0 g |
| $CaCO_3$ | 5,0 g |
| Destilliertes Wasser | 1 l |
| pH | 7,0 |

Zusammensetzung des Produktionsmediums:

| | |
|---|---|
| Mannit | 20,0 g |
| Sojabohnenmehl | 20,0 g |
| Destilliertes Wasser | 1 l |
| pH | 7,2 |

Beim Abernten des Fermenters nach 54 Stunden betrug der pH-Wert der Kulturbrühe 6,46 und das abgesetzte Zellvolumen 18 ml auf 100 ml.

Beispiel V

Kultivierung von *Streptomyces* Y—8340369 (DSM 3278) zur fermentativen Herstellung von Naphthomycin H

Wie Beispiel I bis auf die folgenden Unterschiede:

Zusammensetzung des Impfkulturmediums:

| | |
|---|---|
| Sojabohnenmehl | 15,0 g |
| Stärke | 15,0 g |
| Glucose | 50,0 g |
| $CaCO_3$ | 10,0 g |
| $CoCl_2 \cdot 6\,H_2O$ | 5,0 g |
| Destilliertes Wasser | 1 l |
| pH | 7,0 |

Zusammensetzung des Produktionsmediums:

| | |
|---|---|
| Lösliche Stärke | 20,0 g |
| Glucose | 15,0 g |
| Sojapepton | 3,0 g |
| Pepton | 3,0 g |
| $CaCO_3$ | 2,0 g |
| Maisquellwasser | 2,0 g |
| NaCl | 2,0 g |
| $(NH_4)_2SO_4$ | 0,5 g |
| $CuSO_4 \cdot 5\,H_2O$ | 7,0 g |
| $FeSO_4 \cdot 7\,H_2O$ | 1,0 g |
| $MnCl_2 \cdot 4\,H_2O$ | 8,0 g |

| | |
|---|---|
| $ZnSO_4 \cdot 7\,H_2O$ | 2,0 g |
| Destilliertes Wasser | 1 l |
| pH | 6,7 |

Beim Abernten des Fermenters nach 48 Stunden betrug der pH-Wert 6,78 und das abgesetzte Zellvolumen 18 ml auf 100 ml.

Beispeil VI

Isolierung, Reinigung und Identifizierung von Naphthomycin H

Ca. 90 l Fermentationsbrühe wurden zur Trennung von Mycel und Kulturfiltrat zentrifugiert. Das erhaltene Kulturfiltrat (90 l) wurde mit 2 N NaOH auf einen pH-Wert von 7,0 eingestellt und zweimal mit 30 l Essigsäureethylester extrahiert. Die wäßrigen Schichten wurden verworfen und die vereinigten Essigsäureethylesterextrakte im Vakuum zur Trockene eingedampft. Der erhaltene Rohextrakt betrug ca. 35,8 g. Das erhaltene Mycel (3,9 kg) wurde dreimal mit je 15 l Aceton extrahiert. Die vereinigten Extrakte wurden im Vakuum zur Entfernung des Acetons eingedampft. Die erhaltene wäßrige Schicht wurde mit 2 N NaOH auf einen pH-Wert von 7,0 eingestellt und zweimal mit 5 l Essigsäureethylester extrahiert. Die wäßrigen Schichten wurden verworfen und die vereinigten Extrakte im Vakuum zur Trockene eingedampft. Ca. 16,0 g Rohextrakt wurden erhalten.

36 g Rohextrakt wurden auf eine 3,5 × 49 cm-Kieselgelsäule aufgegeben und nacheinander mit Chloroform und einem Chloroform:Methanol-Gemisch eluiert, in dem die Methanolkonzentration allmählich auf 5% (endgültige Zusammensetzung 95:5) stieg. Man erhielt so 14,6 g einer halbreinen Verbindung, die auf eine 6 × 31 cm große Kieselegelsäule (Maschengröße 200—300) gegeben wurde und unter Druck zunächst mit Benzol und dann mit einem Benzol:Essigsäureethylester-Gemisch bei allmählichem Anstieg der Essigsäureethylester-Konzentration auf 60% (Endzusammensetzung 4:6) eluiert wurde. Dabei erhielt man Fraktion A in einer Menge von etwa 0,19 g und Fraktion B in einer Menge von etwa 1,2 g. Zur weiteren Reinigung wurde Fraktion A auf eine 1,4 × 80 cm-Sephadex LH 20® -Säule gegeben und mit Methanol eluiert, wobei 142 mg einer Verbindung anfielen, die mittels präparativer Dünnschichtchromatographie (Kieselgelplättchen von 0,5 mm Stärke) in einem Gemisch aus Chloroform:Methanol (95:5) gereinigt wurde. Dabie erhielt man 96 mg einer reinen Verbindung, die als das bekannte Antibiotikum Naphthomycin A identifiziert wurde. Dann beschickte man eine 6 × 30 cm-Silica gel-H® -Säule (ohne Bindemittel) als Chromatographiesäule mit der Fraktion B und eluierte mit einem Gemisch aus Chloroform und Methanol (96:4). Dabei erhielt man 650 mg einer halbreinen Substanz, die durch präparative Dünnschichtchromatographie (Kieselgelplättchen von 1 mm Stärke) in einem Gemisch aus Chloroform und Methanol. (95:5) gereinigt wurde und dann aus einem Petroleumäther:Benzol:Essigsäureethylester-Gemisch umkristallisiert wurde, wobei Naphthomycin H in einer Menge von 120 mg anfiel.

Das Naphthomycin H wurde durch chemische-analytische und durch spektroskopische Methoden identifiziert.

Bei der Bestimmung der UV-Absorptionsmaxima wurde Naphthomycin H in einer Konzentration von 10 mg/l verwendet. Das Absorptionsspektrum lag im Bereich von 200 bis 800 nm.

Die Bestimmung der $^1H$ und $^{13}C$ magnetischen Kernresonanzspektren wurde mit einem HX—270 Bruker-Fourier-Transform-Kernresonanzspektrometer bei 270 MHz in $CDCl_3$ durchgeführt.

Die Bestimmung des IR-Spektrums erfolgte in Nujol mit einem Perkin Elmer-IR-Spektrometer P.E. 683, und in KBr mit einem Perkin Elmer-IR-Spektrometer P.E. 521.

Das Massenspektrum wurde mit einem MS—902S,AEI Massenspektrometer unter Verwendung einer FAB (Fast-Atom-Bombardment) Ionenquelle gemessen.

Physikalisch-chemische Eigenschaften von Naphthomycin H
Aussehen: hellgelbes kristallines Pulver
Chemische Formel: $\tilde{C}_{39}H_{44}ClNO_9$
Molkulargewicht: 705 (FAB—MS: $M+H^+ = 706$)
$[\alpha]_D$: +302,93° (c 1,7,$CHCl_3$)
Schmelzpunkt: 150°C (Zers.).

UV-Spektrum
λmax. in
- (a) Methanol: 228, 302 nm
- (b) 0,1N NaOH-Methanol: 216, 236 (sch), 298, 428 nm
- (c) 0,1N NaOH: 240, 298, 425 nm

IR-Spektren:
- (a) in Kbr, C=O-Bereich: 1667, 1626, 1600 und 1577 $cm^{-1}$
- (b) in Nujol (vgl. Fig. 1)

NMR-Spektren:

Die Signale der nicht zum Triensystem gehörenden olefinischen Protonen werden folgendermaßen zugeordnet: Die beiden Doppel-Dubletts bei 5,47 und 5,63 ppm gehören zu einer trans-disubstituierten Doppelbindung (J 16, 17= 15 Hz). Das Dublett bei 5,93 ppm zeigt eine "long range"-Kopplung mit einer Methylgruppe (2,02 ppm, J~1,5 Hz), eine vicinale Kopplung mit einem Methinproton (2,7 ppm, J =10 Hz) und wird deshalb HC(21) zugeordnet. Das Signal für HC(13) erscheint als ein Verbreitertes Triplett bei 6,72 ppm. Kopplungskonstanten des Triensystems im Naphthomycin H (J 2,3 = 11,5 J 4,5, = 11, J 6,7 = 15 Hz) zeigen, daß C(2)=C(3) und C(4)=C(5) eine Z-Geometrie besitzen, während die andere Doppelbindung C(6)=C(7) eine E-Geometrie besitzt (vgl. die Struktur des Naphthomycins H in Formel I).

Tabelle I: $^1$HN—NMR-Entkopplungsexperimente am Naphthomycin H (270 MHz, $CDCl_3$)[a,b]

| Einstrahlung [ppm] | Beobachtetes Signal δ ppm H(X) | Umgewandelt in /J(Hz)[c] | Zuordnung H(X) |
|---|---|---|---|
| HC(3) [6,98] | 6,32 dd (1H) | d/~11 | HC (4) |
| | 6,04 d (1H) | s | HC (2) |
| HC(13) [6,72] | 2,32 m (2H) | u | $H_2C$ (14) |
| | 1,72 d (3H) | s | $H_3C$—C (12) |
| HC(6) [6,52] | 6,32 dd (1H) | d/~11 | HC (5) |
| | 5,54 dd (1H) | d/~10 | HC (7) |
| HC(2) [6,04] | 6,95 dd (1H) | d/11 | HC (3) |
| HC(21) [5,94] | 2,70 m (1H) | dq/3,7 | HC (20) |
| | 2,04 d (3H) | s | $H_3C$—C (22) |
| HC(15) [4,04] | 5,62 dd (1H) | d/15 | HC (16) |
| | 2,32 m (2H) | u | $H_2C$ (14) |
| HC(9) [3,57] | 3,14 dd (1H) | d/17 | $H_2C$(10) |
| | 2,62 dd (1H) | d/17 | $H_2C$(10) |
| | 2,32 m (1H) | u | HC (8) |
| $H_3C$—C(8) [1,22] | 2,32 m (1H) | u | HC (8) |
| $H_3C$—C(18) [0,97] | 2,22 m (1H) | dd/10+10 | HC (18) |
| $H_3C$—C(20) [0,82] | 2,70 m (1H); | dd/3+10 | CH (20) |

a) Kopplungskonstanten in Hz: 2/3 = 11,5; 3/4 = 11; 4/5 = 11; 6/7 = 15; $10_{a/b}$ = 17; 16/15 = 7,5; 21/20 = 10; 22 $CH_3$/21 = 1,5.
b) Es bestehen 5 austauschbare Protonen 2,65 (OH), 3,65 (2 × OH), 8,0 (S, NH), 9,78 (HO—C(25))
c) u = Signalform wegen unvollständiger Entkopplung nicht eindeutig zu erkennen.

In der nachfolgenden Tabelle II wird das Vorhandensein von 39 Kohlenstoffatomen im Naphthomycin H bestätigt.

Tabelle II: $^{13}C$ NMR des Naphthomycins H in $CDCl_3$ (67,9 MHz) in ppm

C = O Bereich: Keton-C-Atome 203,5; 201,6
Chinon-C-Atome 178,6; 178,1
Amid-C-Atome 165,0

$sp^2$-Bereich: 161,2; 147,2; 142,5; 141,6; 138,0; 137,6; 136,8; 136,7; 135,9; 135,5; 134,5; 133,8; 133,3; 132,5; 131,2; 126,5; 123,4; 121,4; 121,0; 119,9

$sp^3$-Bereich: (CH—O, CH—, $CH_2$, $CH_3$—): 76,3; 73,3; 71,8; 45,2; 41,7; 40,6; 36,4; 33,7; 17,4; 16,4; 16,2; 12,4; 11,2; 10,8

Löslichkeit: Löslich in Methanol, Aceton, Essigsäureethylester und Chloroform und schwach löslich in Hexan, Petroleumäther und wäßrigem Alkali. DSC (dünnschichtchromatographische) Daten: Naphthomycin H besitzt die in nachfolgender Tabelle III gezeigten $R_f$-Werte (im Vergleich mit anderen Naphthomycinen an Kieselgelschichten unter Verwendung verschiedener Laufmittelsysteme bestimmt).

Tabelle III: DSC-Daten bei Verwendung von 0,2 mm Silikagel 60 $F_{254}$ (Merck)-Schichten auf Aluminiumfolie.

| Laufmittelsystem | $R_f$-Wert | | |
|---|---|---|---|
| | Naphthomycin | | |
| | A | B | C |
| EtOAc | 0,64 | 0,53 | 0,46 |
| EtOAc: MeOH (98:2) | 0,48 | 0,44 | 0,41 |
| Benzol: EtOAc (2:8) | 0,56 | 0,38 | 0,28 |
| EtOAc: Essigsäure (200:1) | 0,57 | 0,47 | 0,41 |
| EtOAac: $H_2O$: Ameisensäure (100:30:2,5, obere Phase) | 0,74 | 0,64 | 0,63 |

Aus den obigen Ergebnissen in Verbindung mit dem massenspektrometrischen Fragmentierungsmuster wird ersichtlich, daß Naphthomycin H eine neue antibiotisch wirksame Verbindung mit der in Formel I dargestellten Struktur ist.

Es wurde die antimikrobielle Wirksamkeit von Naphthomycin H im Vergleich mit der der Naphthomycine A und B bestimmt. Die Ergebnisse werden angegeben als Zonendurchmesser (in Millimeter) der Inhibitionszone, erzeugt durch die Gegenwart der Naphthomycinverbindung in Form einer Lösung von 1 mg/ml auf einer 6 mm Filterpapierscheibe auf Agar, das den Testorganismus enthält, in der nachfolgenden Tabelle IV.

Tabelle IV:

| Mikroorganismus | Zonendurchmesser (mm) Naphthomycin (1 mg/ml) | | |
|---|---|---|---|
| | H | A | B |
| *Staphylococcus aureus* 209 P | 17 | 14 | 15 |
| *Streptococcus faecalis* | 12 | 9 | 10 |
| *Sarcina lutea* | 18 | 12 | 16 |
| *Bacillus subtilis* | 15 | 9 | 12 |
| *Alcaligenes faecalis* | 12 | — | 10 |
| *Candida albicans* | 18 | 11 | 16 |
| *Penicillium italicus* | 16 | 14P | 15 |
| *Aspergillus niger* | 17/21 | 13 | 14/18 |
| *Saccharomyces cerevisiae* | 10 | — | 10 |
| *Fusarium nivale* | 15 | — | 20 |

P = partielle Zone

Naphthomycin H ist also wirksam gegen grampositive Bakterien und Pilze und kann als Antibiotikum bei der Behandlung von Pilz- und Staphylococceninfektionen eingesetzt werden.

Für die Verwendung der erfindungsgemäßen Verbindung als Antibiotikum kommt eine Dosiseinheit von 12,5—50 mg/kg (p.o.) und die folgende Tagesdosierung in Betracht:

1. Tag (2x) 25 — 100 mg/kg (p.o.)
2. Tag (1x) 12,5 — 50 mg/kg (p.o.)

Für die topische Anwendung gegen Pilzinfektionen werden z.B. Salben oder Lösungen mit 10—20 mg/ml benutzt.

**Patentansprüche für die Vertragsstaaten: BE CH DE FR GB IT LI NL**

1. Naphthomycin H der Formel I

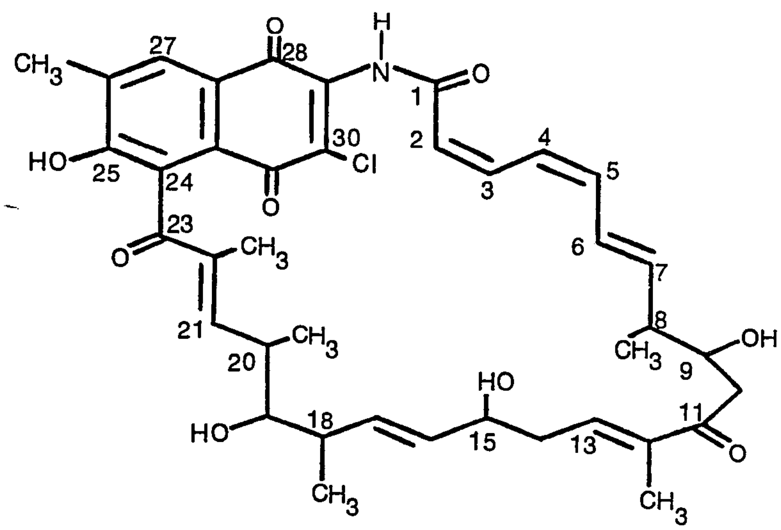

2. Verfahren zur Herstellung von Naphthomycin H gemäß Anspruch 1, dadurch gekennzeichnet, daß der Mikroorganismus Streptomyces DSM 3278 in üblicher Weise unter aeroben Bedingungen in Gegenwart eines Nährmediums fermentiert wird und die gebildete Verbindung aus der Kulturflüssigkeit

und dem Mycel durch Extraktion mit organischen Lösungsmitteln und anschließend durch chromatographische Methoden in üblicher Weise isoliert wird.

3. Verfahren gemäß Anspruch 2, dadurch gekennzeichnet, daß der Mikroorganismus Streptomyces DSM 3278 in einem Kohlenstoff- und Stickstoffquellen sowie anorganische Nährsalze und gegebenenfalls Spurenelemente enthaltenden Nährmedium bei einer Temperatur von 24—40°C und einem pH von 6,0 bis 8,0 fermentiert wird, die gebildete Verbindung aus dem Kulturfiltrat und dem Mycel mit einem Ester, einem niederen Alkanol, einem Keton oder Chloroform extrahiert wird und aus dem Extrakt durch Eindampfen und anschließende chromatographische Reinigung das Naphthomycin H isoliert wird.

4. Arzneimittel, gekennzeichnet, durch einen Gehalt an einer Verbindung gemäß Anspruch 1.

5. Verwendung einer Verbindung gemäß Anspruch 1 zur Herstellung eines Arzneimittels mit antibiotischen Eigenschaften.

**Patentansprüche für den Vertragsstaat: AT**

1. Verfahren zur Herstellung von Naphthomycin H der Formel I,

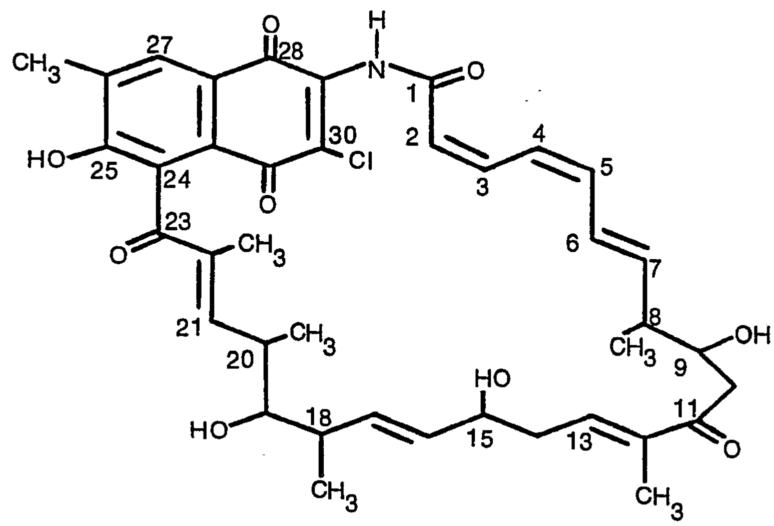

dadurch gekennzeichnet, daß der Mikroorganismus Streptomyces DSM 3278 in üblicher Weise unter aeroben Bedingungen in Gegenwart eines Nährmediums fermentiert wird und die gebildete Verbindung aus der Kulturflüssigkeit und dem Mycel durch Extraktion mit organischen Lösungsmitteln und anschließend durch chromatographische Methoden in üblicher Weise isoliert wird.

2. Verfahren gemäß Anspruch 2, dadurch gekennzeichnet, daß der Mikroorganismus Streptomyces DSM 3278 in einem Kohlenstoff- und Stickstoffquellen sowie anorganische Nährsalze und gegebenenfalls Spurenelemente enthaltenden Nährmedium bei einer Temperatur von 24—40°C und einem pH von 6,0 bis 8,0 fermentiert wird, die gebildete Verbindung aus dem Kulturfiltrat und dem Mycel mit einem Ester, einem niederen Alkanol, einem Keton oder Chloroform extrahiert wird und aus dem Extrakt durch Eindampfen und anschließende chromatographische Reinigung das Naphthomycin H isoliert wird.

3. Verwendung einer Verbindung gemäß Anspruch 1 zur Herstellung eines Arzneimittels mit antibiotischen Eigenschaften.

**Revendications pour les Etats contractants: BE CH DE FR GB IT LI NL**

1. Naphthomycine H de formule I

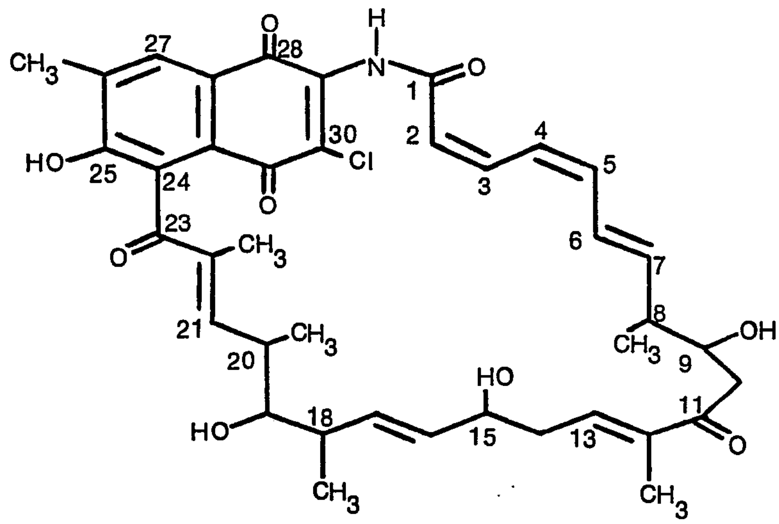

2. Procédé de préparation de la napththomycine H conformément à la revendication 1, caractérisé en ce que l'on fait fermenter le microorganisme Streptomyces DSM 3278 de manière habituelle en conditions aérobies en présence d'un milieu nutritif et en ce que l'on isole le composé formé du liquide de culture et du mycelium par extraction avec un solvant organique et ensuite de manière usuelle par des méthodes chromatographiques.

3. Procédé selon la revendication 1, caractérisé en ce que l'on met à fermenter le microorganisme Streptomyces DSM 3278 dans un milieu de culture contenant des sources de carbone et d'azote ainsi que des sels nutritifs minéraux et éventuellement des oligoéléments à une température de 24 à 40°C et à un pH de 6,0 à 8,0, en ce que l'on extrait le composé formé du filtrat de culture et du mycelium avec un ester, un alcool inférieur, une cétone ou du chloroforme et en ce que l'on isole la naphthomycine H par évaporation et purification ultérieure par chromatographie.

4. Médicament caractérisé en ce qu'il contient un composé selon la revendication 1.

5. Utilisation d'un composé selon la revendication 1 pour la préparation d'un médicament ayant des propriétés antibiotiques.

**Revendications pour l'Etat contractant: AT**

1. Procédé de préparation de naphthomycine H de formule I

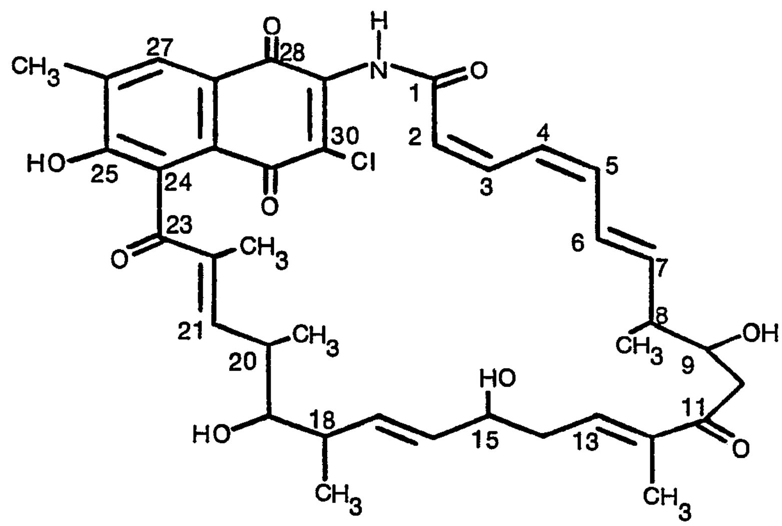

caractérisé en ce que l'on fait fermenter le microorganisme Streptomyces DSM 3278 de manière habituelle en conditions aérobies en présence d'un milieu nutritif et en ce que l'on isole le composé formé du liquide de culture et du mycelium par extraction avec un solvant organique et ensuite de manière usuelle par des méthodes chromatographiques.

2. Procédé selon la revendication 1, caractérisé en ce que l'on met à fermenter le microorganisme Streptomyces DSM 3278 dans un milieu de culture contenant des sources de carbone et d'azote ainsi que des sels nutritifs minéraux et éventuellement des oligoéléments à une température de 24 à 40°C et à un pH de 6,0 à 8,0, en ce que l'on extrait le composé formé du filtrat de culture et du mycelium avec un ester, un alcool inférieur, une cétone ou du chloroforme et en ce que l'on isole la naphthomycine H par évaporation et purification ultérieure par chromatographie.

3. Utilisation d'un composé selon la revendication 1 pour la préparation d'un médicament ayant des propriétés antibiotiques.

**Claims for the Contracting States: BE CH DE FR GB IT LI NL**

1. Naphthomycin H of the formula I

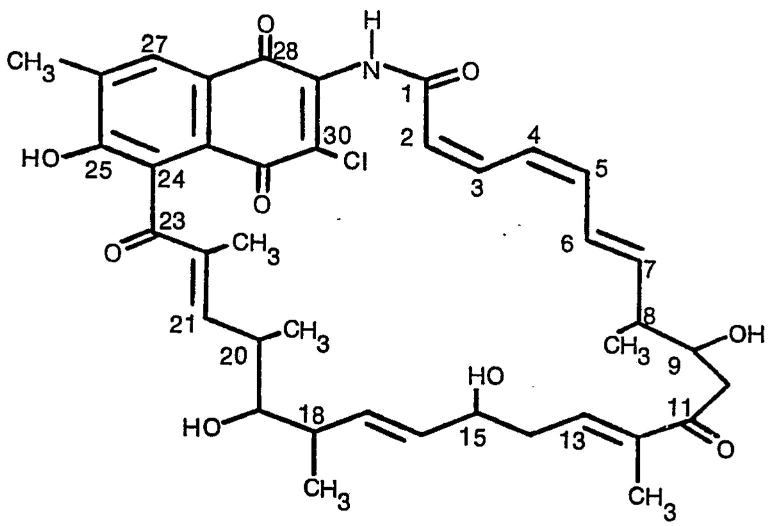

2. A process for the preparation of naphthomycin H as claimed in claim 1, which comprises fermentation of the microorganism Streptomyces DSM 3278 in a customary manner under aerobic conditions in the presence of a nutrient medium, and the compound which is formed being isolated in a customary manner from the culture liquid and the mycelium by extraction with organic solvents followed by chromatographic methods.

3. The process as claimed in claim 2, wherein the microorganism Streptomyces DSM 3278 is fermented in a nutrient medium containing sources of carbon and of nitrogen together with inorganic nutrient salts and, where appropriate, trace elements, at a temperature of 24—40°C and a pH of 6.0 to 8.0, the compound which is formed is extracted from the culture filtrate and the mycelium using an ester, a lower alkanol, a ketone or chloroform, and the naphthomycin H is isolated from the extract by evaporation followed by chromatographic purification.

4. A medicament which contains a compound as claimed in claim 1.

5. The use of a compound as claimed in claim 1 for the preparation of a medicament having antibiotic properties.

**Claims for the Contracting State: AT**

1. A process for the preparation of naphthomycin H of the formula I

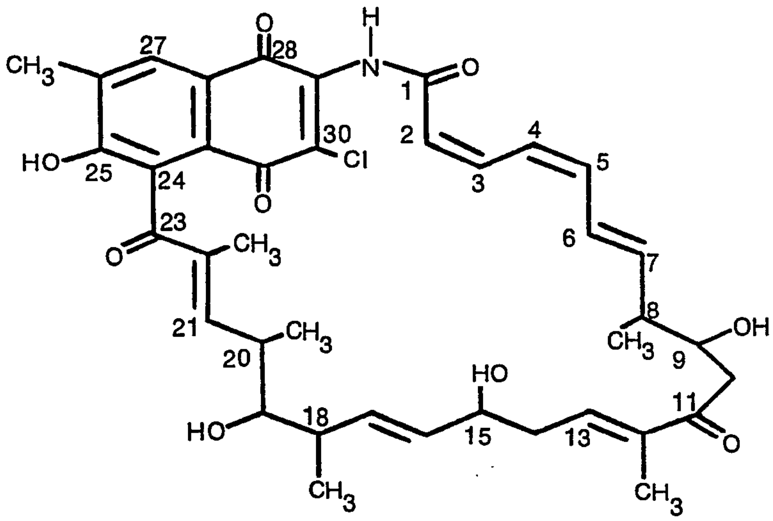

which comprises fermentation of the microorganism Streptomyces DSM 3278 in a customary manner under aerobic conditions in the presence of a nutrient medium, and the compound which is formed being isolated in a customary manner from the culture liquid and the mycelium by extraction with organic solvents followed by chromatographic methods.

2. The process as claimed in claim 1, wherein the microorganism Streptomyces DSM 3278 is fermented in a nutrient medium containing sources of carbon and of nitrogen together with inorganic nutrient salts and, where appropriate, trace elements, at a temperature of 24—40°C and a pH of 6.0 to 8.0, the compound which is formed is extracted from the culture filtrate and the mycelium using an ester, a lower alkanol, a ketone or chloroform, and the naphthmoycin H is isolated from the extract by evaporation followed by chromatographic purification.

3. The use of a compound as claimed in claim 1 for the preparation of a medicament having antibiotic properties.